# EUROPEAN PATENT SPECIFICATION

(11) **EP 2 859 085 B1**
(45) Date of publication and mention of the grant of the patent: **13.09.2017**
(21) Application number: 13801175.4
(22) Date of filing: 05.06.2013
(51) Int. Cl.: C12M 1/16, C12M 1/06, C12M 1/00, C12M 1/12

(54) **SOLID STATE BIOREACTOR ADAPTED FOR AUTOMATION**
FESTKÖRPERBIOREAKTOR FÜR AUTOMATION
BIORÉACTEUR À SEMI-CONDUCTEUR APPROPRIÉ POUR L'AUTOMATISATION

(30) Priority: 06.06.2012 US 201261656175 P; 03.08.2012 US 201261679176 P
(43) Date of publication of application: 15.04.2015
(73) Proprietor: Novozymes Bioag A/S, 2880 Bagsvaerd (DK); Novozymes Biologicals, Inc., Salem, VA 24153 (US)
(72) Inventor: ANDERSEN, Claus, DK-3520 Farum (DK); KORSHOLM, Lars, Salem, Virginia 24153 (US); REZAEI, Farzaneh, Salem, Virginia 24153 (US); CHANG, Felicia, Roanoke, Virginia 24015 (US); SAADAT, Angie P., Roanoke, Virginia 24018 (US)
(74) Representative: Rasmussen, Preben
(86) International application number: PCT/US2013/044312
(87) International publication number: WO 2013/184800

(56) References cited:
- EP-A1- 2 186 876
- EP-B1- 1 794 280
- WO-A1-2010/032260
- US-A- 4 087 327
- US-A1- 2010 203 626
- US-A1- 2010 304 472
- US-B1- 6 620 614
- US-B1- 6 620 614
- US-B2- 7 476 534
- DATABASE WPI Week 201250 Thomson Scientific, London, GB; AN 2012-J17807 XP002752717, -& CN 202 272 865 U (GUANGZHOU FU QUAN BIOTECNOLOGY CO. LTD.) 13 June 2012 (2012-06-13)
- DATABASE WPI Week 201164 Thomson Scientific, London, GB; AN 2011-L54980 XP002752718, -& CN 201 942 676 U (HUNAN SHUN XIN BIO-TECHNOLOGY CO. LTD.) 24 August 2011 (2011-08-24)

## Description

### FIELD OF THE INVENTION

The invention relates to the field of solid state bioreactors and fermentation using the reactors.

### BACKGROUND OF THE INVENTION

Solid state fermentation involves the cultivation of selected microorganisms, such as fungi, on solid typically granular growth media. Products produced by solid state fermentation include enzymes, nutritional food additives, antibiotics and insecticidal spores, among others.

Prior attempts at production-scale solid state fermentation in closed bioreactors, such as those disclosed in U.S. Patent Nos. 6,620,614 and 7,476,534, have relied on internally stacked tray levels. However, a shortcoming of such designs is that each level can experience a different microenvironment within the vessel during the fermentation cycle. In addition, such designs require active cooling for each level increasing the complexity of the reactor as well as increasing the costs of operating the reactor during the fermentation cycle. Lastly, these reactors are not well-suited for automation because the reactor vessel must be partially disassembled to remove the tray levels upon completion of the fermentation cycle.

What is needed and provided by the present invention are improved production-scale, solid state bioreactors that provide consistent and repeatable fermentation conditions throughout the culture media while being adapted for automation and modular scalability.

### SUMMARY OF THE INVENTION

The present invention provides an improved solid state bioreactor suitable for automation while at the same time facilitating uniform fermentation with reduced operating costs. Unlike the prior bioreactors, the reactor vessel of the invention does not require an active cooling apparatus increasing energy consumption but instead relies on evaporative cooling.

In one embodiment, the invention provides a production-scale solid state bioreactor with a reactor vessel, including:
a top wall having an upper surface that may be at least substantially planar and a lower surface that may be at least substantially planar;
a bottom wall having an upper surface that may be at least substantially planar and a lower surface that may be at least substantially planar, the bottom wall defining the base of the reactor vessel;
one or more side walls connecting the top wall and the bottom wall, the top, bottom and one or more side walls thereby collectively defining an interior compartment of the reactor vessel, a vertical height from bottom to top and an expansive horizontal dimension,
   wherein a plurality of apertures is formed in one or more of the walls (top, bottom and side walls) such as in the one or more side walls;
at least one reversibly-openable closure connected to a wall in which an aperture is formed, the closure sized and configured to reversibly seal the aperture; and
a horizontally-oriented perforated plate member disposed inside the interior compartment of the reactor vessel at a level between the bottom wall and the top wall, the plate member having an upper side and a lower side, wherein the plate member comprises at least one perforated plate and wherein if the plate member includes more than one plate, each plate is disposed at least substantially at the same level whereby no plate substantially horizontally overlaps another plate. A reversibly-openable closure may be provided and connected to each of the apertures. In one embodiment, the reactor vessel is provided with a water sprayer means in fluid communication with the interior compartment. In another embodiment, the reactor vessel is provided with an agitator means above the perforated plate member.

A related embodiment of the invention provides a production-scale solid state bioreactor with a reactor vessel including:
a top wall having an upper surface that may be at least substantially planar and a lower surface that may be at least substantially planar;
a bottom wall having an upper surface that may be at least substantially planar and lower surface that may be at least substantially planar, the bottom wall defining the base of the bioreactor;
one or more side walls connecting the top wall and the bottom wall, the top, bottom and one or more side walls thereby collectively defining an interior compartment a vertical height from bottom to top and an expansive horizontal dimension,
   wherein a plurality of apertures is formed in one or more of the walls (top, bottom and side walls), such as in the one or more side walls;
at least one reversibly-openable closure connected to a wall in which an aperture is formed, the closure sized and configured to reversibly seal the aperture; and
   a horizontally oriented drawer disposed between the bottom wall and the top wall, the drawer including:
      a base panel comprising a horizontally-oriented perforated plate member having an upper side and a lower side, wherein the plate member comprises at least one perforated plate, and wherein if the plate member includes more than one plate, each plate is disposed at least substantially at the same level and no plate substantially horizontally overlaps another plate,
      two side panels, a back panel and a front panel, the front panel sealable with an aperture in the side wall of the bioreactor in which the drawer is insertable when the drawer is fully inserted therein (when the drawer is closed). In addition to the aperture sealable by the drawer, a reversibly-openable closure may be provided and connected to each of the apertures.

A further embodiment of the invention provides a production-scale solid state bioreactor system, including:
a plurality of production-scale solid state bioreactors according to any of the embodiments or variations thereof described herein; and
a fermentation station including a plurality of air-providing lines and air exhaust lines, the fermentation station and plurality of bioreactors mutually adapted to operably and reversibly connect each of the plurality of bioreactors to at least one air-providing line and at least one air exhaust line.

Still further embodiments of the invention provide methods for preparing the bioreactor and bioreactor systems of the invention for the solid-state fermentation of microoorganisms therein, methods for conducting the solid-state fermentation of microorganisms within the bioreactors and methods for recovering and purifying resulting solid-state fermentation product(s).

Additional features, advantages, and embodiments of the invention may be set forth or apparent from consideration of the following detailed description, drawings, and claims. Moreover, it is to be understood that both the foregoing summary of the invention and the following detailed description are exemplary and intended to provide further explanation without limiting the scope of the invention as claimed.

### BRIEF DESCRIPTION OF THE DRAWINGS

FIG. 1 is a perspective view with a partial cut out of the solid state bioreactor of the invention equipped with external componentry.
FIG. 2 is a perspective view with a partial cut out of the solid state bioreactor vessel of the invention configured with a water sprayer means.
FIG. 3 is a perspective view with a partial cut out of the solid state bioreactor vessel of the invention configured with an agitator means.
FIG. 4 is a top cross-sectional view of the embodiment shown in FIG. 3 taken along line 4-4.
FIG. 5 is a side cross-sectional view of the embodiment shown in FIG. 3 taken along line 5-5.
FIG. 6 is a perspective view with a partial cut out of the solid state bioreactor vessel of the invention configured with an alternative embodiment of the agitator means.
FIG. 7 is a top cross-sectional view of the embodiment shown in FIG. 6 taken along line 7-7.
FIG. 8 is a side cross-sectional view of the embodiment shown in FIG. 6 taken along line 8-8.
FIG. 9 is an alternative cross-sectional view of the embodiment shown in FIGS. 6 and 8 taken along line 9-9.
FIG. 10 is a perspective view with a partial cut out of an alternative cylindrical embodiment of the solid state bioreactor vessel of the invention configured with the agitator means.
FIG. 11 is a side view of the embodiment of the embodiment shown in FIG. 10.
FIG. 12 a top cross-sectional view of the embodiment shown in FIG. 11 taken along line 12-12
FIG. 13 is a side cross-sectional view of the embodiment shown in FIG. 10 taken along line 13-13.

### DETAILED DESCRIPTION OF THE INVENTION

The invention provides reusable production-scale solid state bioreactors designed to facilitate and maximize the automation of solid state fermentation processes while maintaining aseptic conditions and transfer of materials. The invention also provides automated solid state fermentation systems that include the bioreactors as scalable modules.

The invention provides solutions to several of the obstacles typically associated with surface fermentation:

First, insufficient cooling (and respiration) inside the growth media at higher bed heights typically restricts the fermentation process in prior solid state fermentation bioreactor designs. A high bed of growth media is desirable from an economic perspective because it lowers the number of trays that need to be handled. The bioreactors of the present invention advantageously avoid the problems typically associated with using high (i.e., deep or thick) media beds as a result of their air flow configuration. In accordance with the invention, air for cooling and respiration is forced through the bed of growth media in a selected direction that can be alternated, if desired. Through the use of evaporative cooling, the reactor vessel does not require active cooling equipment inside the reactor vessel as typically found in prior multi-level bioreactor systems. As a result, the reactor vessel of the invention can omit an active cooling system thereby reducing the complexity of the reactor vessel and reducing operating costs due to energy consumption by cooling equipment.

Second, low moisture content typically restricts the fermentation process. Evaporation of water from solid state growth media typically contributes to cooling the media but at the expense of drying it out which restricts the fermentation process. The bioreactors of the present invention overcome this issue by permitting water to be added frequently, and preferably aseptically, during the fermentation cycle. Thus, a high evaporative cooling effect can be achieve while at the same time the moisture content is kept at an optimally high level.

Third, production-scale solid state fermentation using conventional trays or levels is typically very labor intensive and not well-suited to automation. The bioreactors of the present invention, in contrast, can be of such size that each bioreactor individually replaces many conventional trays with a single perforated plate member. In addition, bioreactors of the present invention are adapted for handling by standard heavy duty industrial robots in an automated environment.

Fourth, contamination of sensitive product may reduce yields and throughput in conventional solid state fermentation apparatuses. The risk of contamination of the product inside a conventional tray is increased every time a process is applied to a tray, or if the tray is transported. The bioreactor designs of the present invention eliminate or minimize the risk of contamination when configured in an aseptic configuration. With the present invention, the growth media may be steam sterilized within the bioreactor compartment and, due to the aseptic design, the whole enclosure remains sterile except for inoculation with the desired microorganism.

Fifth, the bioreactors of the present invention also provide worker safety advantages. Worker exposure to microorganisms, such as fungi, is a risk when performing conventional tray or bag-based solid state fermentations. The fully enclosed design of the bioreactors of the present invention, when configured in an aseptic configuration, virtually eliminates this risk by preventing environmental escape of the microorganism. Through their automation, the bioreactor systems of the present invention may also eliminate the risk of competitive strain injury that may otherwise be present in non-automated large-scale tray and bag-based fermentation processes. For example, with the multi-level reactors of the prior art, the reactor vessel must be opened upon completion of the fermentation cycle followed by each tray being removed by a technician. Such a design provides multiple opportunities for injury to the technician.

Sixth, the bioreactors of the present are reusable and decrease waste in comparison to disposable solid state fermentation bags and trays known in the art.

The present invention is further described with respect to the appended figure as follows.

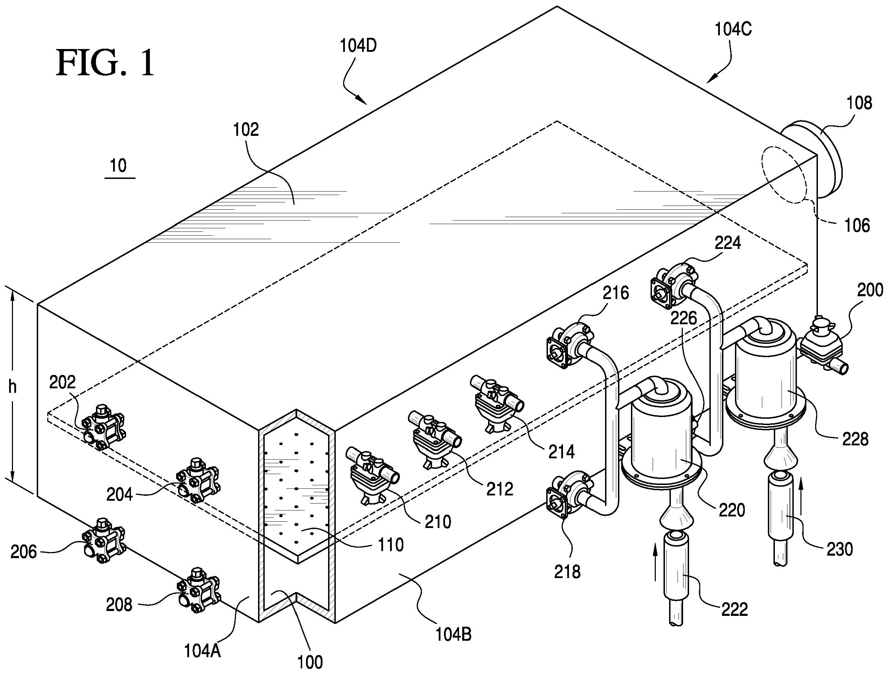

FIG. 1 shows a partial cut-away, perspective view of a production-scale, solid state bioreactor embodiment of the invention with an aseptic configuration. The bioreactor may be one of a plurality of such bioreactors that are components of an at least partially automated and/or mechanized production-scale bioreactor system. As shown in FIG. 1, reactor vessel 10 including a lower, base portion (or wall) 100 and an upper top portion (or wall) 102 and side walls 104A-D to form a reactor vessel shaped as a rectangular box defining an interior space (or compartment) for receipt of the fermentation growth media. Although reactor vessel 10 is depicted as a rectangular box, it will be apparent to those skilled in the art that reactor vessel 10 can be in a variety of shapes.

Referring back to FIG. 1, reactor vessel 10 includes a perforated plate member 110 disposed horizontally inside the vessel at a position intermediate (i.e., between) lower, base portion 100 and upper, base portion 102 bisecting the interior compartment (not labeled) into respective upper and lower compartments (not labeled). Plate member 110 is joined to sidewalls 104A-D by any conventional means and can be sealed along the edges to sidewalls 104A-D to maximize air being forced through the perforations of plate member 110. In one embodiment, perforated plate 110 can be sealed to sidewalls 104A-D by a continuous weld. In another embodiment (not shown in FIG. 1), reactor vessel 10 may comprise upper and lower housings with perforated plate 110 disposed between the housing sections whereby perforated plate 110 extends past sidewalls 104A-D.

Perforated plate member 110 is preferably disposed inside reactor vessel 10 at a position proximal to lower, base portion 100 and distal from upper, base portion 102. The positioning of plate member 110 proximal to lower, base portion 100 maximizes the available volume of the upper compartment since it is on top of perforated plate member 110 that solid state growth media will be dispersed. Representative examples of growth media include, but not limited to, rice, rice bran, cracked wheat, pearl barley, feed barley, and barley flake.

While the distance between lower, base portion 100 and perforated plate member 110 is variable, they should be sufficiently spaced to maximize air distribution and to allow access for a cleaning device (not shown). For example, lower, base portion 100 and perforated plate member 110 should be spaced apart by at least two (2) inches. Of course, as the size of the reactor vessel increases, the distance between lower, base portion 100 and perforated plate member 110 may also increase.

Although not depicted in FIG. 1, perforated plate member 110 may be formed from multiple perforated plates joined along their edges in a horizontal direction to effectively provide the same functionality as a single perforated plate. In such a configuration, the plates should not overlap which would reduce the flow of air through the perforated plate member and possibly lead to different microenvironments within the solid state growth media. In one embodiment, perforated plate member 110 is a single perforated plate.

As shown in FIG. 1, access to the volume of the compartment above perforated plate member 110 (i.e., the upper compartment) is provided by an aperture, access port 106, disposed in one of side walls 104A-D (e.g., sidewall 104C) of reactor vessel 10. While only one access port is depicted in FIG. 1, it will be clearly apparent to those skilled in the art that a plurality of access ports can be provided for greater access to the interior of reactor vessel 10. Access port 106 is sealable by plug 108 during use of the fermentation reactor. Plug 108 and corresponding aperture (access port 106) is preferably mutually adapted to reversibly seal the aperture to facilitate access to the interior of reactor vessel 10. For example, a reversible seal or single-use seal can be provided by interlocking screw threads permitting the plug to be reversibly screwed into the aperture. Alternatively, a reversible seal can be provided by reversibly clamping the plug to the aperture. Any means known in the art, such as a valve, can be used in accordance with the invention.

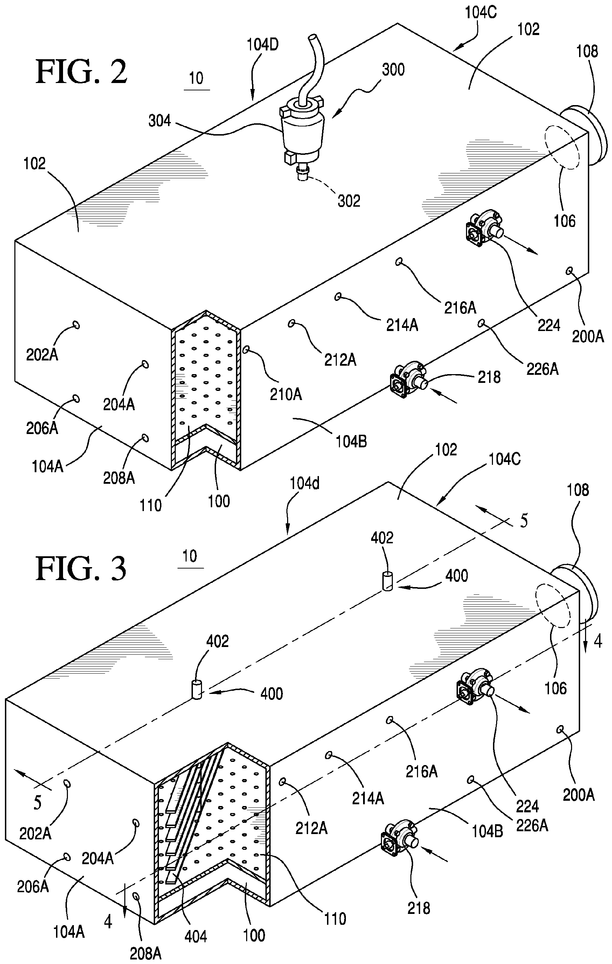

Still referring to FIG. 1, a number of other apertures can be formed in the side walls of vessel 10 that are connected to (i.e., in fluid communication) with valves or other reversibly, openable hardware. For example, as depicted in FIG. 1, reactor vessel 10 includes drain valve 200 connected to an aperture (shown in FIG. 2 as 200A) in a lower corner of the bioreactor. Reactor vessel 10 can also include upper compartment valves 202 and 204 in fluid communication with apertures openings (shown in FIG. 2 as 202A and 204A) for attaching wash lines to the upper compartment of the bioreactor thereby allowing water to be sprayed onto the inner surfaces for cleaning and rinsing purposes. Similarly, reactor vessel 10 can also include lower compartment valves 206 and 208 in fluid communication with apertures openings (shown in FIG. 2 as 206A and 208A) for attaching wash lines to the lower compartment of the bioreactor. For example, high pressure cleaning nozzles may be inserted into the upper and lower compartments using these aperture openings.

Likewise, as shown in FIG. 1, reactor vessel 10 is be provided with additional upper compartment, valves 210, 212 and 214 connected to apertures (shown in FIG. 2 as 210A, 212A and 214A) opening into the compartment above perforated plate member 110. Valves 210, 212 and 214 may be designated a variety of functions such as being connected to lines for adding water, adding inoculant and for taking samples. In addition, valves 210, 212 and 214 if adapted for water delivery may be connected to spray or mister nozzles configured to deliver spray or mist into the upper and/or lower compartments of reactor vessel 10.

The bioreactor can also include air inlet lines (shown in FIG. 1 as valves 216 and 218), which are valved air inlet lines connected to apertures openings (not shown in FIG. 1) leading into the upper and lower compartments, respectively. Air inlet valves 216 and 218 are in fluid communication with a common tube portion (not labeled) that is in fluid communication with air inlet filter 220. Air inlet filter 220 provides sterile air for aseptic condition within reactor vessel 10. The other side of air inlet filter 220 is attached a valve component (not labeled) sized and configured to reversibly mate with a fixed ball valve adaptor 222 of an air providing line at a fermentation station at which the bioreactor is used. Fixed ball valve adaptor 222 allows for a quick connection to the reactor docking station. In general, the air inlet hardware of the bioreactor and the air providing line hardware of the fermentation station will be mutually adapted to reversibly connect to each other. This external componentry ensures that filtered air is provided to reactor vessel 10 minimizing the risks of unwanted contamination of the fermentation growth media.

Still referring to FIQ. 1, the bioreactor can also include air outlet lines (shown in FIG. 1 as valves 224 and 226), which are valved air outlet lines connected to aperture openings (not shown in FIG. 1) leading from the upper and lower compartments, respectively. Air outlet valves 224 and 226 are in fluid communication with a common tube portion (not labeled) that is in fluid communication with air outlet filter 228. Air outlet filter 228 filters the air being exhausted from reactor vessel 10 for the safety of the reactor technicians. In practice, a sterile air outlet filter is also needed on the exhaust line in order to ensure that aseptic conditions are kept inside the reactor vessel 10. The other side of air outlet filter 228 is attached a valve component (not labeled) sized and configured to reversibly mate with a fixed ball valve adaptor 230 of an air exhaust line at a fermentation station at which the bioreactor is used. In general, the air outlet hardware of the bioreactor and the air exhaust line hardware of the fermentation station will be mutually adapted to reversibly connect to each other.

Air inlet valves 216 and 218 may be individually controllable, and likewise air outlet valves 224 and 226 may be individually controllable. These valves may be electrically or pneumatically controllable. The controllability of the valves permits the direction of air flow with respect to perforated plate member 110 to be selected and reversed as desired or needed. Stated otherwise, the air flow through reactor vessel 10 can be in the direction from above to below (i.e., flowing from the upper compartment to the lower compartment) or from below to above (i.e., flowing from the lower compartment to the upper compartment).

Still referring to FIG. 1, one or more of the aperture openings can also be mounted with additional air inlets for feeding air to reactor vessel 10. As shown on FIG. 1, reactor vessel 10 has 2 air inlet openings/valves 216,218. Likewise, exhaust air can leave reactor vessel 10 via openings/valves 224,226. By equipping reactor vessel 10 with 2 or more openings in communication with the upper compartment and 2 or more openings in communication with the lower compartment, additional air can be forced through the perforated plate member 110 and the substrate/media bed on top of perforated plate member 110. The air can be blown in both directions; top to bottom or bottom to top. For example, any one of valves 202, 204, 206 or 208 can be adapted to provide additional air to reactor vessel 10 and to alter the direction of the air flow. Such an alternative configuration is beneficial because air is required for several processes inside the reactor such as fermentation/air respiration, cooling, heating, and adjusting moisture levels.

In addition, as shown in FIG. 1, the height (h) of the reactor vessel of the present invention is the distance measured in the direction transverse (i.e., perpendicular) to the horizontal plane of perforated plate member 110. The width and length are measured in the directions transverse to the height. Although FIG. 1 shows a rectangular box configuration for reactor vessel 10, the profile of the bioreactor transverse to its height may be any shape. For example, the reactor vessel 10 is not limited to the rectangular shape depicted in FIG. 1 but can be oval, circular, square, triangular, trapezoidal, kidney-shaped, and so. As shown in FIG. 1, the maximum dimension of the bioreactor transverse to its height (i.e., in the horizontal dimension) is greater than the height (both dimensions measured by the main walls forming the compartment of reactor vessel 10 and not the exterior-joined componentry). In accordance with the present invention, the reactor vessel should have an aspect ratio defined as the ratio of the maximum dimension of the bioreactor transverse to its height to the height of the reactor vessel may be greater than 1.0 where both dimensions are measured by the main walls forming the compartment of the reactor vessel and does not included exterior joined componentry. The aspect ratio of the reactor vessel of the present invention may, for example, be greater than 1.0, greater than 1.5, greater than 2.0, greater than 2.5, greater than 3.0, greater than 3.5, greater than 4.0, greater than 4.5 or greater than 5.0. The aspect ratios for the reactor vessel of the present invention may, for example, range from 1.0 to 10.0, 1.5 to 10.0, 2.0 to 10.0, 2.5 to 10.0, 3.0 to 10.0, 3.5 to 10.0, 4.0 to 10.0, 4.5 to 10.0, and 5.0 to 10.0.

Referring to FIG. 2, provided is a perspective view with a partial cut out of the solid state bioreactor vessel of the invention configured with a water sprayer means. In contrast to FIG. 1, reactor vessel 10 is shown in a non-aseptic configuration with all the external componentry removed with the exception of air inlet valve 218 and air outlet valve 224. In accordance with the invention, one or more of aperture openings can be reversibly sealed since the bioreactor of the invention in operation just requires a single aperture opening to each of the upper and lower compartments. As shown in Fig. 2, reactor vessel 10 includes water sprayer means 300 adapted to upper, base portion 102 and in fluid communication with the upper compartment of reactor vessel 10. Water sprayer means includes nozzle 302 disposed within the upper compartment of reactor vessel 10. Nozzle 302 is in fluid communication with sterile water filter 304 to provide aseptic conditions within reactor vessel 10. While water sprayer means 300 is centrally positioned on upper, base portion 102, water sprayer means 300 or a plurality of water sprayer means 300 can be positioned anywhere on reactor vessel 10 so long as the sprayer means is connected (i.e., in fluid communication with) the upper compartment. Examples of nozzles to be used in accordance with the invention are the C-Series hydraulic atomizing nozzles commercially available from BEX Incorporated, located in Ann Arbor, Michigan.

In another embodiment of the invention, as shown in FIG. 3, reactor vessel 10 is adapted with at least one agitator means 400. Agitator means 400 allows the fermentation growth media to be mixed before, during or after the fermentation cycle. A benefit of the agitator means is to promote a more uninform mixture of the fermentation growth media. This in turn reduces the likelihood of different microenvironments forming within media that can occur with prior reactor designs. As shown in FIG. 3, agitator means 400 can be a rotor shaft 402 extending through upper, base portion 102 towards lower, base portion 100. Rotor shaft 402 has a plurality of rotors 404 adapted thereto whereby rotation of rotor shaft 402 ensures rotation of rotors 404 to agitate the fermentation growth media. While agitator means 400 is depicted as extending in a vertical direction into the upper compartment of reactor vessel 10, agitator means 400 can be positioned in any location in the upper compartment as long the fermentation growth media can sufficiently be mixed. Rotation of rotor shaft 402 can be achieved by any means known in the art such as with the use of an electric or pneumatic motor. Likewise, the movement of rotors 404 can be synchronous or asynchronous depending on the dimensions of rotors 404. Lastly, while agitator means 400 is depicted as a rotor, agitator means 400 can be any suitable structure (such as an auger or a screw) for mixing the fermentation growth media.

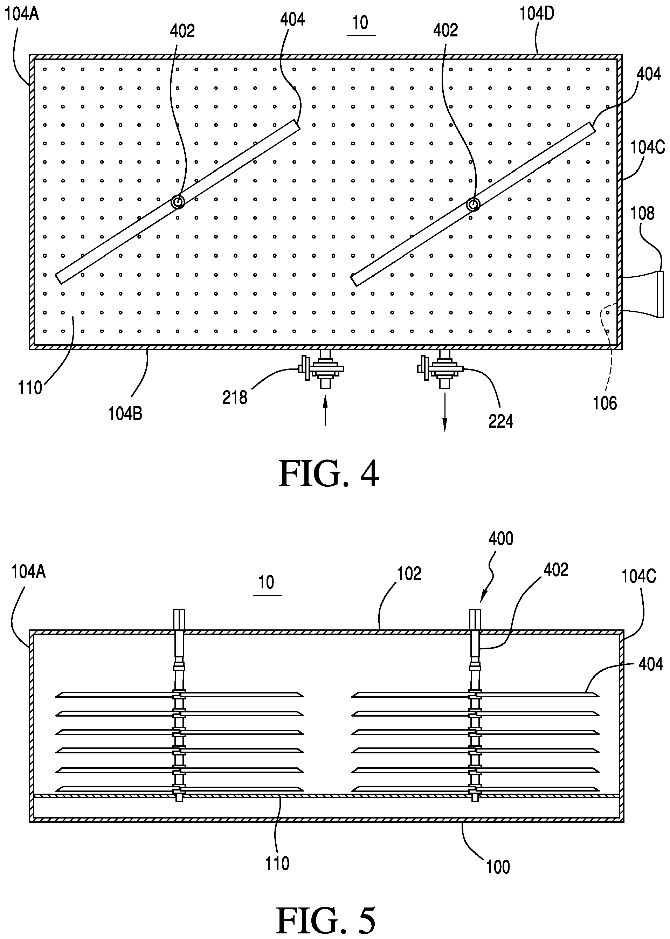

FIGS. 4 and 5 depict top and side cross-sectional views of the reactor vessel 10 of FIG. 3. As shown in FIG. 5, rotors 404 are spaced apart thereby preventing unwanted contact between rotors sets. However, rotors 404 can also be configured to intersect without contact to ensure a uniform agitation of the fermentation growth media.

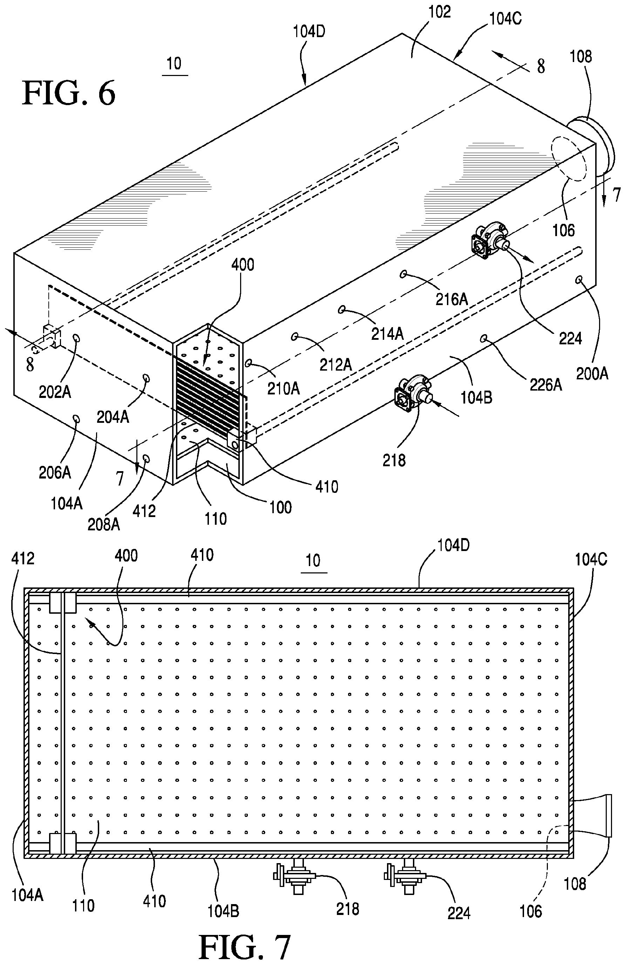

Referring to FIG. 6, provided is a perspective view with a partial cut out of the solid state bioreactor vessel of the invention configured with an alternative agitator means. As shown in FIG. 6, reactor vessel 10 is adapted with agitator means 400 in the form of a mechanical sledge 412 reciprocating within the upper compartment along sledge guide rails 410. The reciprocating (i.e., back and forth) movement of sledge 412 facilitates greater mixing of the fermentation growth media allowing for a more uniform or homogenous fermentation environment.

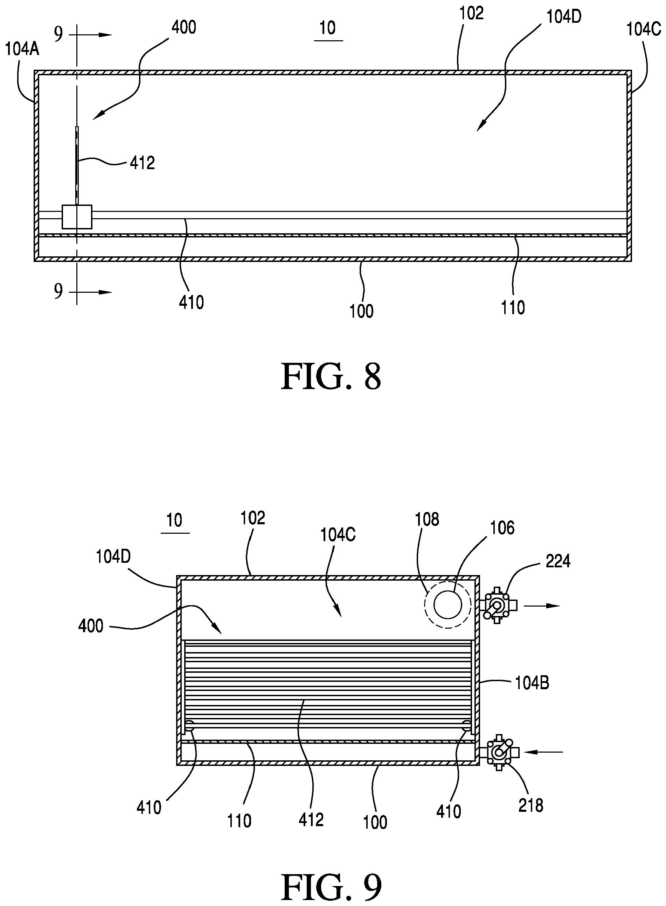

FIGS. 7-9 show alternative views of reactor vessel 10 equipped with the alternative agitator means 400 depicted in FIG. 6. As shown in FIG. 7, reactor vessel 10 includes a pair of sledge guide rails 410 extending along parallel to side walls 104B,104D whereby sledge 412 adapted to guide rails 410 extends the entire width (i.e., horizontal dimension) of reactor vessel 10. FIGS. 8 and 9 show sledge 412 does not extend the entire vertical dimension of the upper compartment of reactor vessel 10. Sledge 412 merely needs to extend the depth of the fermentation growth media to ensure sufficient mixing to promote a more uniform microenvironment. However, as will be apparent to those skilled in the art, guide rails 410 and sledge 412 can provided in the upper compartment of reactor vessel 10 in numerous different configurations to ensure sufficient mixing of the fermentation growth media.

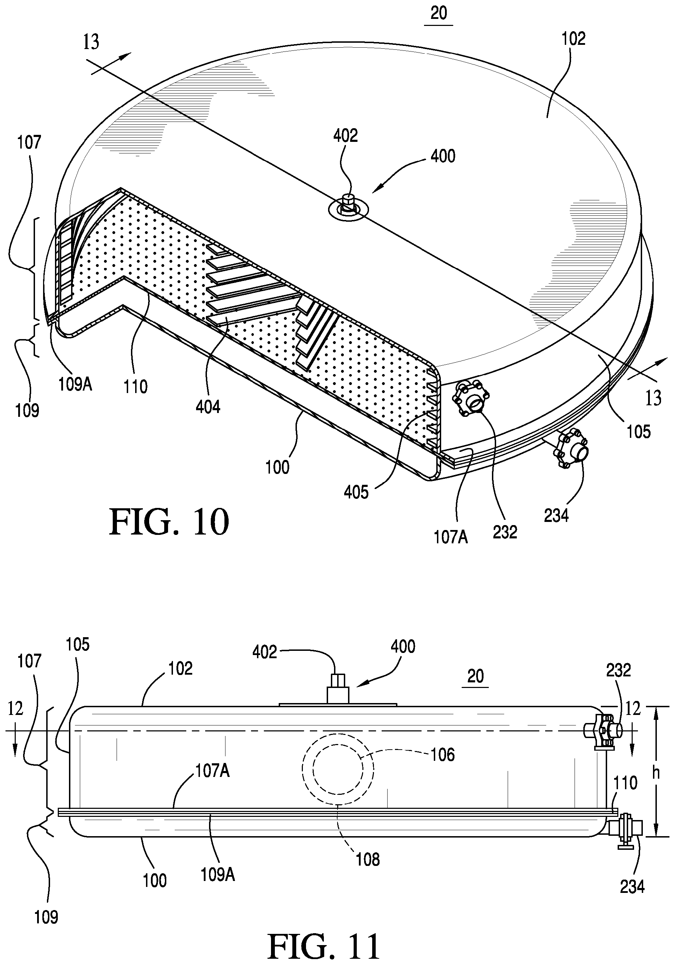

Referring to FIG. 10, provided is a perspective view with a partial cut out of the solid state bioreactor vessel 20 where reactor vessel 20 is in a cylindrical configuration. In accordance with the invention, reactor vessel 20 while in a cylindrical configuration, has a horizontal dimension significantly greater than its vertical dimension thereby maintaining an aspect ratio as described above. Reactor vessel 20 includes vertically spaced lower, base portion 100 and upper, base portion 102 with an arcuate (i.e., curved) sidewall 105 positioned between base portions 100,102. In accordance with the invention, reactor vessel 20 includes perforated plate member 110 (shown as a single plate) extending horizontally the entire interior dimension of the vessel. As a result, perforated plate member 110 bisects the interior compartment (not labeled) into upper and lower compartments (not labeled). Likewise, perforated plate member 110 is positioned proximal to lower, base portion 100 to provide the upper compartment with a greater interior volume relative to the lower compartment.

Still referring to FIG. 10, reactor vessel 20 (unlike reactor vessel 10) is adapted with perforated plate member 110 extending past sidewall 105 whereby the housing of reactor vessel 20 is bisected into a two-part housing including an upper housing 107 and a lower housing 109. As shown in FIG. 10, upper and lower housings 107,109 include exterior flanges 107A,109A extending circumferentially at the open ends of their respective housings. Perforated plate member 110 is positioned intermediate flanges 107,109A whereby housings 107A,109A and perforated plate 110 are joined in an abutting relationship via bolts, clamps or the like. Because of the two housings, reactor vessel 20 can be disassembled if desired. In the alternative, housings 107,109 and perforated plate member 110 can be welded together if disassembly is not required.

Reactor vessel 20 can be configured in a similar fashion to reactor vessel 10. For example, reactor vessel 20 is adapted with agitator means 400, which as shown in FIG. 10, includes rotor shaft 402 extending vertically through upper, base portion 102 into the upper compartment. Rotor shaft 402 is adapted with a plurality of rotors 404 in a manner analogous to reactor vessel 10. Upper housing 107 of reactor vessel 20 is also provided with optional rotor guides 405 within the interior of the housing. Although not shown, stators can be mounted on optional rotor guides 405 to improve the functionality of agitator means 400. Reactor vessel 20 is also adapted with upper and lower housing valves 232,234 in fluid communication with (i.e., connected to) the upper and lower compartments (not labeled) via upper and lower aperture openings (not shown in FIG. 10). Upper and lower housing valves 232,234 allow reversible access for air, water and any other fluid medium to be introduced into the interior of reactor vessel 20. Although not shown in FIG. 10, reactor vessel 10 can also be provided with a plurality of aperture openings for access in manner analogous to reactor vessel 10.

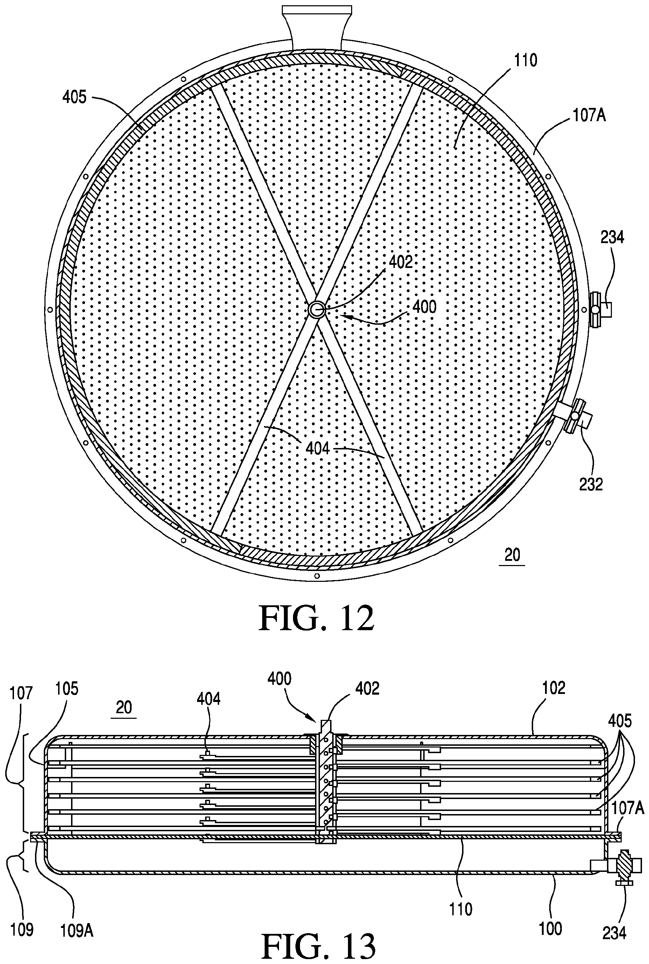

FIGS. 11-13 show alternative views of reactor vessel 20 equipped with the agitator means 400 depicted in FIG. 10. As shown in FIG. 11, reactor vessel 20 is also provided with an access port 106 in fluid communication with the upper compartment and is sealable with plug 108 or a suitable valve (not shown). In manner analogous to reactor vessel 10, reactor vessel 20 can be adapted with a plurality of access ports to facilitate access to the upper compartment. Rotors 404, as shown in FIG. 12, can extend the full horizontal dimension of reactor vessel 20. Referring to FIG. 13, rotors 404 are supported with optional rotor guides 405. FIG. 13 also shows rotor shaft 402 being optionally extended through perforated plate member 110 to provide additional structural support to agitator means 400.

The walls of the reactor vessels 10,20 and perforated plate member 110 disposed therein is preferably constructed of metal such as, but not limited to, stainless steel and aluminum. Alternately, non-metallic materials such as, but not limited to, metallocene polymers or carbon fiber may also be used. The bioreactors of the present invention may be constructed in any manner known in the art. For example, the bioreactor can be assembled with bolting, riveting, welding, and adhesive joining. Practically, joining of any kind or any combination thereof can be used to construct the bioreactor chamber (i.e., reactor vessel). It should be readily understood that in the case where any panel or surface meets another, a gasket or other sealing means may be used to seal the junction. The chamber of a bioreactor such as that shown in FIGS. 1 and 10 can be constructed by metal forming and welding stainless steel panels together and inserting and welding the perforated plate in place before welding the chamber closed. The various apertures can be formed by any method such as, but not limited to, drilling or laser cutting or any method known in the art. Perforated plate member 110 may be similarly formed from a sheet of metal or other material by drilling or punching or any suitable means. The size of the perforations in the perforated plate is selected so that standard granular solid state fermentation growth media, such as rice bran, does not easily pass or does not pass at all. The various components that communicate with the apertures may be joined thereto by any means such as, but not limited to, screw connection thereto or welding thereto in the case of components that need not be reversibly joined.

### Example of Preparation and Operation of Bioreactor for Fermentation:

A bioreactor unit according to the invention, such as that shown in FIG. 1 may be sterilized using steam. The entire bioreactor may, for example, be placed in an autoclave for such sterilization. After filling the bioreactor with growth media, it may be sterilized using steam supplied through one or more of the apertures in the walls of the bioreactor. After this sterilization, the bioreactor may delivered to a fermentation station with its various valves (and drawer if present) in a closed state to prevent external contamination of the interior contents of the bioreactor. The connection sides of the relevant external componentry such as the various valves may then be steam sterilized in place after connection to external piping at a fermentation station. Then the valves on the bioreactor and on the piping of the station may be opened to permit aseptic flow of air, water, liquids, inoculant, etc.

In the following example of a procedure, valves are considered to be closed if not operated.
1. Growth media is filled via access port 106 into the upper interior volume of the bioreactor and the port is then sealed with plug 108.
2. Steam may be supplied through valves 216, 218, 224 and 226 from steam providing lines (similarly attached as lines 222 and 230), whereby the growth media, inner surfaces and air filters 220 and 228 are sterilized together. Alternatively, the entire bioreactor unit may be sterilized within an autoclave.
3. Water/liquids/inoculate may then be added via valves 210 or 212. The bioreactor may be in an upside-down (base facing upwards) position during this operation.
4. After the addition of water/liquids/inoculant, the bioreactor can be rotated and/or shaken in order to mix the components. The bioreactor may be in an upside-down position during this operation or agitator means 400 may be activated in lieu of shaking and rotating of the reactor vessel. The bioreactor is then returned to an upright position if necessary.
5. Excess water/liquids/inoculant accumulated in the bottom of the bioreactor may be drained via valve 200 if desired or necessary at any point.
6. Air for respiration and/or cooling is added via air inlet line 222; and the air/gas leaves via air exhaust line 230 - these spring-loaded ball valves are not mounted on the bioreactor itself, but on the rack positions in the fermentation station so that the valves operably engage the air inlet and outlet connections of the bioreactor when the bioreactor is placed in the fermentation station. If valve 218 and 224 are open (and 216 and 226 closed), the air enters the compartment below perforated plate member 110 and will pass through the growth media bed in an upwards direction. If valves 216 and 226 are open (and 218 and 224 closed) the opposite air direction happens (in a downwards direction). Changing the air direction regularly prevents the growth media from drying out in localized areas of the media bed, thereby providing a more even moisture content during fermentation. This improves microbial growth inside the bed, and facilitates better cooling by evaporation of water from the growth media. When the moisture content falls below a desired value then water/liquid may be added. For example, the bioreactor can be provided with water sprayer means 300 to maintain the moisture of the growth media at an optimum level or to clean the bioreactor after the fermentation cycle is completed.
8. Lumps that may form in the bed of growth media may be broken up by placing the bioreactor on a (heavy duty) vibration station. Alternatively, the growth media is mixed with agitator means 400 by turning on the agitator thereby replacing the need for a vibration station.
9. Samples may be removed from the bioreactor during fermentation via valve 214.
10. Solid product may be emptied out through access port 106. The bioreactor may for example be tilted in a vertical side-on-end position to pour the contents of its upper volume out of port 106 and/or the contents may be removed therefrom using a utensil and/or vacuum.
11. The interior of the bioreactor may, for example, be washed by spray nozzles that are inserted via valved wash apertures 202, 204, 206 and 208

### Description of Support Systems and Stations Employed with the Bioreactors

### Chambers and Racks:

A bioreactor according to the invention may be placed in a rack position sized and configured to receive the bioreactor at a fermentation station. Each rack position may have connection members, such as, but not limited to ball valves, that are adapted to connect the bioreactor to an air supply line and an air exhaust line of an Air System (see description below). Water lines and the like may also be connected to the bioreactor when it is in the rack position of the fermentation station.

### Air System:

Air for respiration, cooling, and/or drying inside the compartments may be conditioned centrally in a few main air handling units (AHU). The air may be conditioned to desired temperature, humidity, pressure and cleanliness required for a given product and process phase. A duct pipe system designed for low pressure loss may be provided to distribute the air to the bioreactor connection points at each rack position.

### Transportation of Bioreactors:

Transport of compartments between various stations and rack positions may be automated. Transportation between stations may be performed using standard automated guided vehicles (AGV's). Particular operations, such as placing a bioreactor in a rack position and removing a bioreactor therefrom, may be performed using standard industrial handling robot.

### Operations of Solid State Fermentation System and Equipment Therefor:

Some of the following unit operations may optionally be combined into one or several work station (a machine):
Filling of growth media into the reactor vessel; performed by a filling machine.

Loading and unloading of trolley for autoclave; performed by a handling robot.

Adding of water/liquid/inoculant and soaking; performed by dedicated machines. Inoculant may, for example, be fed from a standard seed culture apparatus.

Draining of water/liquid/inoculate; performed by a dedicated machine.

Turning / gentle mixing; performed by a handling robot or by the agitator.

Lump break / shaking / harsh mixing; performed by a heavy duty vibration machine or by the agitator.

Change of air flow direction inside the reactor vessel; performed by actuation of valves connected to apertures on a bioreactor removably installed at the fermentation station.

Weight control; an electronic scale.

Inspection and sample taking; may be performed manually and/or be automated.

Emptying of solids including products from inside the compartments; performed by a handling robot or semi-manually inside an isolator.

Draining of liquid product (extraction) from inside the compartment; may be performed manually or be automated.

### Management of the Production Process:

The production process within the system may be computer controlled and monitored. Every compartment may be provided with a machine-readable identification (ID), such as a machine-readable ID tag, for tracking within the system. Some or all of the work stations, AGV's or robots may be provided with an ID reader, such as an ID tag reader. In this manner, the system can track the position and progress of every bioreactor unit.

Standard production recipes may be downloaded into the computerized control system. The computerized control system in response to a request can plan and execute all orders to the various robots and automated sub-systems regarding transport, handling and operation.

Without limitation, the following embodiments and variations thereof are provided by the invention:
One embodiment of the invention provides a production-scale solid state bioreactor with a reactor vessel including:
   a top wall having an upper surface that may be at least substantially planar and a lower surface that may be at least substantially planar;
   a bottom wall having an upper surface that may be at least substantially planar and a lower surface that may be at least substantially planar, the bottom wall defining the base of the bioreactor;
   one or more side walls connecting the top wall and the bottom wall, the top, bottom and one or more side walls thereby collectively defining an interior compartment, a vertical height from bottom to top and an expansive horizontal dimension,
      wherein a plurality of apertures is formed in one or more of the walls, such as in the one or more side walls;
   at least one reversibly-openable closure connected to a wall in which an aperture is formed, the closure sized and configured to reversibly seal the aperture; and
   a horizontally-oriented perforated plate member disposed, such as fixed or suspended, inside the compartment at a level between the bottom wall and the top wall, the plate member having an upper side and a lower side, wherein if the plate member includes more than one plate, each said plate is disposed at least substantially at the same level and no plate substantially horizontally overlaps another plate. A reversibly-openable closure may be provided and connected to each of the apertures.

A related embodiment of the invention provides a production-scale solid state bioreactor with a reactor vessel including:
a top wall having an upper surface that may be at least substantially planar and a lower surface that may be at least substantially planar;
a bottom wall having an upper surface that may be at least substantially planar lower surface that may be at least substantially planar, the bottom wall defining the base of the bioreactor;
one or more side walls connecting the top wall and the bottom wall, the top, bottom and one or more side walls thereby collectively defining a compartment having an interior, a vertical height from bottom to top and an expansive horizontal dimension,
   wherein a plurality of apertures is formed in one or more of the walls, such as in the one or more side walls; and
at least one reversibly-openable closure connected to a wall in which an aperture is formed, the closure sized and configured to reversibly seal the aperture; and
   a horizontally oriented drawer disposed between the bottom wall and the top wall, the drawer including:
      a base panel comprising a horizontally-oriented perforated plate member having an upper side and a lower side, wherein if the plate member includes more than one plate, each plate is disposed at least substantially at the same level and no plate substantially horizontally overlaps another plate,
      two side panels, a back panel and a front panel, the front panel sealable with an aperture in the side wall of the bioreactor in which the drawer is insertable when the drawer is fully inserted therein (when the drawer is closed). In addition to the aperture sealable by the drawer, a reversibly-openable closure may be provided and connected to each of the apertures. The drawer may, for example, be slideably mounted on rails in the bioreactor or grooves formed in the walls of the bioreactor.

The perforated plate member in the embodiments preferably completely separates the interior of the compartment into a portion above the plate to define an upper compartment and into a portion of the compartment below the plate to define a lower compartment. The two portions of the compartment at least predominantly, such as only, in fluid communication with each other through the perforations in the perforated plate member. The perforated plate member may consist of one or more perforate plates at the same level joined in a non-overlapping, abutting relationship.

In one variation of the embodiments, at least one of the apertures (aside from the aperture in which the drawer fits) opens into the compartment above the level at which the perforated plate member is disposed and at least one of the apertures opens into the compartment below the level at which the perforated plate member is disposed. At least some of the plurality of apertures, such as all of the apertures, may be formed in the one or more side walls. Some or no apertures may be formed in the top wall and bottom wall.

It is preferred that the maximum horizontal dimension of the compartment is greater than the vertical height of the compartment. Thus, the bioreactor compartment may have an aspect ratio greater than one.

As will be apparent to those skilled in the art, the horizontal area of the perforated plate member, whether one or more plates, is where growth media is placed. As a result, the horizontal area of the bioreactors themselves can be quite large. The area of the perforated plate member may, for example, have an area (on one side) of at least 1.0 m², such as in the range of 1.0-5.0 m², such as about or equal to 1.0 m², equal to or about 1.5 m², equal to or about 2.0 m², equal to or about 2.5 m² or equal to or about 3.0 m². The term "about" as used herein means within a range of ±5% with respect to a specified value. The height of a bioreactor interior compartment of the invention may, for example, be in the range of 50-500 cm, such as 100-400 cm or 150-300 cm. The thickness of the walls forming the reactor vessel of a bioreactor according to the invention will generally be small in comparison to the overall dimensions of the bioreactor or the perforated plate therein. For example, the walls of the bioreactor may be in the range of 0.25 cm to 2.0 cm thick. In one variation, a bioreactor according to the invention and/or the perforated plate (or plates collectively) therein is at least substantially rectangular with horizontal dimensions of equal to or about 1.0m on one side and about 2.0 m to about 2.5 m, such as equal to or about 2.0 m or equal to or about 2.5 m, on the other side, the height of the bioreactor being smaller than either of the horizontal dimensions and, for example, being in the range of 50-500 cm, such as 100-400 cm or 150-300 cm. A production-scale bioreactor according to the invention with these dimensions is able to replace at least 50-100 standard 2.0kg disposable plastic solid state fermentation bags (that typically require radiation sterilization).

As shown in FIG. 1, the bioreactor may further include an air inlet filter operably communicating with (connected to) at least one of the apertures. The bioreactor may further include an air outlet filter operably communicating with (connected to) at least one of the apertures. At least one of the apertures of the bioreactor vessel may be operably and reversibly connected to an air inlet line. At least one of the apertures of the bioreactor vessel may be operably and reversibly connected to an air outlet line. The filters are preferably sterile filters that block the passage of microorganisms, such as 0.25 micron or smaller pore filters, for example, 0.2 micron or smaller pore filters. Thus, both inward and outward contamination can be prevented during transport of the bioreactor and during cultivation of selected organisms in the bioreactor.

While not shown in FIGS. 1-13, the bioreactors of the invention may further include a machine-readable identification, such as a machine readable identification tag of any kind that identifies a particular bioreactor unit. Suitable machine readable identification tags include, for example, barcodes (used in conjunction with a barcode reader) and RFID tags (used in conjunction with an RFID tag reader).

Bioreactors may also be provided with one or more lamps or other light sources that insert into an aperture in a wall, such as one disposed above the level of the at least one perforated to provide illumination within the upper volume of the bioreactor.

Very high solid phase growth media beds may advantageously be used in the bioreactors of the present invention. These beds may, for example, be at least 10 cm, at least 15 cm, at least 20 cm, up to or about 40cm and/or up to or about 50 cm high. The height of the media bed may, for example, be in the range of 10 cm to 50 cm, or 10 cm to 40 cm. The invention also provides any of the bioreactor embodiments described herein further including the bed of solid state growth media, such as granular solid state growth media, such as rice bran, loaded within to any of the heights specified. In bioreactor embodiments of the invention that do not have a drawer, the perforated plate member and the side walls of the bioreactor retain the growth media in the upper compartment above the perforated plate member. In bioreactor embodiments of the invention that have a drawer, the height of the back panel, side panels and front panel of the drawer are selected to accommodate a desired range of media bed heights. For example, a drawer with sides having a height of equal to or at least 50 cm is suitable for media bed heights of up to 50 cm.

In a further embodiment, invention provides a production-scale solid state bioreactor system, including:
a plurality of production-scale solid state bioreactors according to any of the embodiments or variations thereof described herein, such as at least 10 of said bioreactors, at least 100 of said bioreactors, at least 500 of said bioreactors or at least 1000 of said bioreactors, or at least 2000 of said bioreactors; and
a fermentation station including a plurality of air-providing lines and air exhaust lines, the fermentation station and plurality of bioreactors being mutually adapted to operably and reversibly connect each of the plurality of bioreactors to at least one air-providing line and one air exhaust line. Each of the bioreactors of the plurality of bioreactors may be the same design or at least substantially the same design, but in any case operable (cooperating) with the fermentation station.

The fermentation station may include a plurality of substations, each substation sized and configured to receive one of the production-scale solid state bioreactors. At least some of the substations may, for example, be arranged in a stacked configuration.

The system may further include at least one mechanized handler sized and configured to insert the bioreactors into the substations and remove the bioreactors from the substations. The mechanized handler may be automated and/or under the control of a computerized control system.

In addition to the fermentation station, the system may, for example, include one or more of the following stations: a solid state culture media filling and mixing station; an inoculation station; a wash station; a sterilization/autoclave station; and a harvesting/product removal station. Those skilled in the art will recognize that some station functions such as washing and sterilization could be combined into a single station. Transport of bioreactors between the stations is preferably automated and may be performed using automated guide vehicles (AGVs). The various functions performed at each station may also be at least partially automated and performed using industrial robots. Autoclaves used for sterilization of the bioreactor should be sized and configured to accommodate and sterilize one or more of the bioreactors at one time.

Microorganisms that may be cultivated within the solid-state bioreactors of the invention and using the bioreactor systems of the invention include but are not limited to fungi, such as, for example, anamorphic fungi, and bacteria, such as, for example, thermophilic acidophilic bacterium. Particular fungi that may be cultivated using the bioreactors and bioreactor systems of the invention include but are not limited to: species of *Aschersonia*; *Beauveria*; *Hirsutella*; *Isaria*; *Lecanicillium*; *Metarhizium, Trichoderma*; *Penicillium* and *Nomuraea, including, e.g., Aschersonia aleyrodis*; *Beauveria bassiana*; *Beauveria brognartii*; *Hirsutella thompsonii*; *Isaria spp*.; *Lecanicillium spp*.; *Metarhizium anisopliae*, *Metarhizium spp*.; *Trichoderma spp*.; *Penicillium bilaiae (Penicillium bilaii)* and *Nomuraea rileyi.*

Fermentation products that may be produced using the solid state bioreactors include, for example, alcohols (e.g., ethanol, methanol, butanol); organic acids (e.g., citric acid, acetic acid, itaconic acid, lactic acid, gluconic acid); ketones (e.g., acetone); amino acids (e.g., glutamic acid); gases; antibiotics (e.g., penicillin and tetracycline); enzymes; vitamins; and hormones. Examples of enzymes include pectinases, amylases, glucoamylases, lipases, proteases, xylanase, and cellulases.

Without limitation, the invention also provides the following methods for the preparation of the solid-state bioreactors for aseptic fermentation and for solid-state fermentation of desired microorganisms therewith.

One embodiment of the invention provides a method for solid state fermentation that includes the steps of:
(a) providing a production-scale solid state bioreactor with a reactor vessel according to any of the embodiments or variations thereof described herein;
(b) introducing solid state fermentation growth media into the space above the perforated plate member (i.e., in the upper compartment of the reactor vessel); and
(c) introducing steam into the interior of the reactor vessel via at least one of the apertures to sterilize the bioreactor and the growth media therein. The bioreactor may include a sterile air outlet filter communicating with the interior of the reactor vessel and a sterile air inlet filter communicating with the interior of the reactor vessel. The method of the embodiment may further include the step of: (d) introducing a preselected microorganism desired to be cultured, such as any of those described herein, into the upper compartment after the bioreactor has been sterilized, for example, via one or more apertures. The microorganism is then cultured within the bioreactor for a period of time, such as but not limited to at least one day, at least two days, at least seven days and at least ten days. The fermentation product(s) may then be recovered from the bioreactor and optionally purified.

In a further embodiment of the invention, the invention provides a method for producing a solid state fermentation product that includes the steps of:
providing a plurality of production-scale solid state bioreactors according to any of the embodiments or variations thereof described herein,
in at least one of the plurality of production-scale solid state bioreactors, introducing solid state fermentation growth media into the space above the perforated plate member (i.e., in the upper compartment of the reactor vessel);
in the at least one of the plurality of production-scale solid state bioreactors, introducing into the interior compartment a preselected microorganism desired to be cultured, such as any of those described herein, said microorganism producing a desired solid-state fermentation product; and
culturing the microorganism in the at least one of the production-scale solid state bioreactor to produce the solid-state fermentation product. The period of culturing the microorganism within the bioreactor may be at least one day, at least two days, at least seven days or at least ten days. The plurality of bioreactors may include at least at least 10 of said bioreactors, at least 100 of said bioreactors, at least 500 of said bioreactors or at least 1000 of said bioreactors, or at least 2000 of said bioreactors. The at least one of the plurality of bioreactors may include one, at least two, at least three, at least five or at least ten bioreactors such as but not limited to at least three 10 of said bioreactors, at least 100 of said bioreactors, at least 500 of said bioreactors or at least 1000 of said bioreactors, or at least 2000 of said bioreactors. The method of the embodiment may further include the step of: introducing steam into the interior of the compartment of the at least one of the plurality of production-scale solid state bioreactors between the steps of introducing the solid state fermentation growth media and introducing the preselected microorganism. The method of the embodiment may further include the step of: after the culturing step, recovering the solid-state fermentation product from the at least one of the plurality of solid-state bioreactors.

In either of the aforementioned method embodiments and their variations, culturing the microorganism in the bioreactor(s) may further include maintaining and/or controlling the temperature and/or humidity/water-content at desired or preselected levels within the bioreactor and/or controlling air flow in and out of the bioreactor. Controlling airflow may include controlling the direction of air flow with respect to above and below the perforated plate member and/or changing or alternating said direction of air flow during the culture period.

Either of the aforementioned method embodiments and their variations may further include the steps of (i) drying the solid-state fermentation product or (ii) extracting the solid-state fermentation product. For example, drying may comprise drying within the bioreactor(s) and/or drying after the fermentation product has been recovered from the bioreactor(s). Likewise, drying may comprise spray drying the solid-state fermentation product. Alternatively, drying may also comprise freeze-drying the solid-state fermentation product or by drying on a fluidized bed. Lastly, either of the aforementioned method embodiments and their variations may further include the step of purifying the solid-state fermentation product after removal from the bioreactor(s).

Although the invention has been described in connection with specific preferred embodiments, it should be understood that the invention as claimed should not be unduly limited to such specific embodiments.

## Claims

1. A production-scale solid state bioreactor with a reactor vessel comprising: a top wall having an upper surface and a lower surface; a bottom wall having an upper surface and a lower surface, the bottom wall defining the base of the reactor vessel; one or more side walls connecting the top wall and the bottom wall, the top, bottom and one or more side walls thereby collectively defining an interior compartment, a vertical height from bottom to top and an expansive horizontal dimension, wherein a plurality of apertures is formed in one or more of the walls; at least one reversibly-openable closure connected to a wall in which an aperture is formed, the closure sized and configured to reversibly seal the aperture; and a perforated plate member horizontally-oriented and disposed in the interior compartment of the reactor vessel at a level between the bottom wall and the top wall, the plate member having an upper side and a lower side, wherein the plate member comprises at least one perforated plate and wherein if the plate member includes more than one plate, each plate is disposed at least at the same level and no plate horizontally overlaps another plate, wherein the at least one reversibly-openable closure includes air inlet and air outlet lines which are individually controllable permitting selection and reversal of the direction of air flow with respect to perforated plate member.

2. The production-scale solid state bioreactor of claim 1, wherein the perforated plate member completely separates the interior compartment of the reactor vessel into a portion above the plate member and into a portion below the plate, the two portions of the interior compartment being in communication with each other through the perforations of the perforated plate member.

3. The production-scale solid state bioreactor of claim 2, wherein the perforated plate member consists of one perforated plate.

4. The production-scale solid state bioreactor of claim 1, further comprising a water sprayer means adapted to the reactor vessel and in communication with the interior compartment of the reactor vessel.

5. The production-scale solid state bioreactor of claim 1, further comprising an agitator means disposed in the interior compartment of the reactor vessel at a position above the perforated plate member.

6. The production-scale solid state bioreactor of claim 1, wherein
at least one of the apertures opens into the compartment above the level at which the perforated plate member is disposed; and
at least one of the apertures opens into the compartment below the level at which the perforated plate member is disposed.

7. The production-scale solid state bioreactor of claim 1, wherein the maximum horizontal dimension of the reactor vessel is greater than the vertical height of the reactor vessel.

8. The production-scale solid state bioreactor of claim 1, further comprising an air inlet filter operably communicating with at least one of the apertures.

9. The production-scale solid state bioreactor of claim 1, further comprising an air outlet filter operably communicating with at least one of the apertures.

10. The production-scale solid state bioreactor of claim 1, further comprising a machine-readable identification tag.

11. A production-scale solid state bioreactor system, comprising:
a plurality of production-scale solid state bioreactors according to claim 1;
a fermentation station comprising a plurality of air-providing lines and air exhaust lines, the fermentation station and plurality of bioreactors mutually adapted to operably and reversibly connect each of the plurality of bioreactors to at least one air-providing line and at least one air exhaust line.

12. The production-scale solid state bioreactor system of claim 11, wherein
the fermentation station comprises a plurality of substations, each substation sized and configured to receive one of the plurality of production-scale solid state bioreactors.

13. The production-scale solid state bioreactor system of claim 11, wherein at least some of the substations are arranged in a stacked configuration.

14. The production-scale solid state bioreactor system of claim 11, wherein each of the bioreactors further comprises an agitator means disposed in the interior compartment of the reactor vessel at a position above the perforated plate member.

## Patentansprüche

1. Festphasen-Bioreaktor im Produktionsmaßstab mit einem Reaktorgefäß umfassend: eine obere Wand mit einer oberen Oberfläche und einer unteren Oberfläche; eine Bodenwand mit einer oberen Oberfläche und einer unteren Oberfläche, wobei die Bodenwand die Basis des Reaktorgefäßes definiert; eine oder mehrere Seitenwände, die die obere Wand und die Bodenwand verbinden, wodurch die obere, die Boden- und eine oder mehrere Seitenwände gemeinsam einen inneren Raum, eine vertikale Höhe vom Boden bis zum oberen Ende und eine ausgedehnte horizontale Dimension definieren, wobei eine Vielzahl an Öffnungen in einer oder mehreren der Wände gebildet ist; mindestens einen reversibel öffenbaren Verschluss, der mit einer Wand verbunden ist, in der eine Öffnung gebildet ist, wobei der Verschluss so bemessen und konfiguriert ist, dass er die Öffnung reversibel verschließt; und ein perforiertes Plattenelement, das horizontal orientiert und im inneren Raum des Reaktorgefäßes in einer Höhe zwischen der Bodenwand und der oberen Wand angeordnet ist, wobei das Plattenelement eine Oberseite und eine Unterseite aufweist, wobei das Plattenelement mindestens eine perforierte Platte umfasst, und wobei, wenn das Plattenelement mehr als eine Platte einschließt, jede Platte mindestens in der gleichen Höhe angeordnet ist und keine Platte eine andere Platte horizontal überlappt, wobei der mindestens eine reversibel öffenbare Verschluss Lufteinlass- und Auslassleitungen einschließt, die individuell kontrollierbar sind, was Auswahl und Umkehr der Richtung des Luftstroms bezüglich dem perforierten Plattenelement erlaubt.

2. Festphasen-Bioreaktor im Produktionsmaßstab nach Anspruch 1, wobei das perforierte Plattenelement den inneren Raum des Reaktorgefäßes vollständig in einen Teil oberhalb des Plattenelements und in einen Teil unterhalb der Platte aufteilt, wobei die zwei Teile des inneren Raumes durch die Perforationen des perforierten Plattenelements miteinander in Kommunikation stehen.

3. Festphasen-Bioreaktor im Produktionsmaßstab nach Anspruch 2, wobei das perforierte Plattenelement aus einer perforierten Platte besteht.

4. Festphasen-Bioreaktor im Produktionsmaßstab nach Anspruch 1, weiterhin umfassend einen Wasserspraymitteladapter zum Reaktorgefäß und in Kommunikation mit dem inneren Raum des Reaktorgefäßes.

5. Festphasen-Bioreaktor im Produktionsmaßstab nach Anspruch 1, weiterhin umfassend ein Rührmittel, angebracht im inneren Raum des Reaktorgefäßes in einer Position über dem perforierten Plattenelement.

6. Festphasen-Bioreaktor im Produktionsmaßstab nach Anspruch 1, wobei
mindestens eine der Öffnungen sich in den Raum über der Höhe öffnet, an der das perforierte Plattenelement angebracht ist; und
mindestens eine der Öffnungen sich in den Raum unterhalb der Höhe öffnet, an der das perforierte Plattenelement angebracht ist.

7. Festphasen-Bioreaktor im Produktionsmaßstab nach Anspruch 1, wobei die maximale horizontale Dimension des Reaktorgefäßes größer ist als die vertikale Höhe des Reaktorgefäßes.

8. Festphasen-Bioreaktor im Produktionsmaßstab nach Anspruch 1, weiterhin umfassend einen Lufteinlassfilter, der funktionsfähig mit mindestens einer der Öffnungen kommuniziert.

9. Festphasen-Bioreaktor im Produktionsmaßstab nach Anspruch 1, weiterhin umfassend einen Luftauslassfilter, der funktionsfähig mit mindestens einer der Öffnungen kommuniziert.

10. Festphasen-Bioreaktor im Produktionsmaßstab nach Anspruch 1, weiterhin umfassend eine maschinenlesbare Identifikationsmarkierung.

11. Festphasen-Bioreaktorsystem im Produktionsmaßstab, umfassend:
eine Vielzahl an Festphasen-Bioreaktoren im Produktionsmaßstab gemäß Anspruch 1;
eine Fermentationsstation, umfassend eine Vielzahl an luftbereitstellenden Leitungen und Abluftleitungen, wobei die Fermentationsstation und die Vielzahl an Bioreaktoren gegenseitig angepasst sind, jeden der Vielzahl an Bioreaktoren mit mindestens einer luftbereitstellenden Leitung und mindestens einer Abluftleitung funktionsfähig und reversibel zu verbinden.

12. Festphasen-Bioreaktorsystem im Produktionsmaßstab nach Anspruch 11, wobei
die Fermentationsstation eine Vielzahl an Unterstationen umfasst, wobei jede Unterstation bemessen und konfiguriert ist, einen der Vielzahl an Festphasen-Bioreaktoren im Produktionsmaßstab zu erhalten.

13. Festphasen-Bioreaktorsystem im Produktionsmaßstab nach Anspruch 11, wobei mindestens einige der Unterstationen in einer gestapelten Konfiguration angeordnet sind.

14. Festphasen-Bioreaktorsystem im Produktionsmaßstab nach Anspruch 11, wobei jeder der Bioreaktoren weiterhin ein Rührmittel umfasst, angebracht im inneren Raum des Reaktorgefäßes in einer Position über dem perforierten Plattenelement.

## Revendications

1. Bioréacteur à semi-conducteur à échelle de production avec une cuve de réacteur comprenant : une paroi supérieure ayant une surface supérieure et une surface inférieure ; une paroi inférieure ayant une surface supérieure et une surface inférieure, la paroi inférieure définissant la base de la cuve de réacteur ; une ou plusieurs parois latérales raccordant la paroi supérieure et la paroi inférieure, les parois supérieure, inférieure et les une ou plusieurs parois latérales définissant ainsi collectivement un compartiment intérieur, une hauteur verticale de bas en haut et une dimension horizontale expansive, dans lequel une pluralité d'ouvertures est formée dans l'une ou plusieurs des parois ; au moins une fermeture pouvant s'ouvrir de manière réversible raccordée à une paroi dans laquelle une ouverture est formée, la fermeture étant dimensionnée et configurée pour sceller de manière réversible l'ouverture ; et un élément de plaque perforée orienté horizontalement et disposé dans le compartiment intérieur de la cuve de réacteur à un niveau entre la paroi inférieure et la paroi supérieure, l'élément de plaque ayant un côté supérieur et un côté inférieur, dans lequel l'élément de plaque comprend au moins une plaque perforée et dans lequel si l'élément de plaque comprend plus d'une plaque, chaque plaque est disposée au moins au même niveau et aucune plaque ne recouvre horizontalement une autre plaque, dans lequel la au moins une fermeture pouvant s'ouvrir de manière réversible comprend des conduites d'entrée d'air et de sortie d'air qui peuvent être individuellement commandées, permettant la sélection et l'inversion de la direction de l'écoulement d'air par rapport à l'élément de plaque perforée.

2. Bioréacteur à semi-conducteur à échelle de production selon la revendication 1, dans lequel l'élément de plaque perforée sépare complètement le compartiment intérieur de la cuve de réacteur en une partie située au-dessus de l'élément de plaque et en une partie située au-dessous de la plaque, les deux parties du compartiment intérieur étant en communication entre elles par le biais des perforations de l'élément de plaque perforée.

3. Bioréacteur à semi-conducteur à échelle de production selon la revendication 2, dans lequel l'élément de plaque perforée se compose d'une plaque perforée.

4. Bioréacteur à semi-conducteur à échelle de production selon la revendication 1, comprenant en outre un moyen de pulvérisation d'eau adapté sur la cuve de réacteur et en communication avec le compartiment intérieur de la cuve de réacteur.

5. Bioréacteur à semi-conducteur à échelle de production selon la revendication 1, comprenant en outre un moyen d'agitation disposé dans le compartiment intérieur de la cuve de réacteur dans une position située au-dessus de l'élément de plaque perforée.

6. Bioréacteur à semi-conducteur à échelle de production selon la revendication 1, dans lequel :
au moins l'une des ouvertures s'ouvre dans le compartiment situé au-dessus du niveau auquel l'élément de plaque perforée est disposé ; et
au moins l'une des ouvertures s'ouvre dans le compartiment situé au-dessous du niveau auquel l'élément de plaque perforée est disposé.

7. Bioréacteur à semi-conducteur à échelle de production selon la revendication 1, dans lequel la dimension horizontale maximum de la cuve de réacteur est supérieure à la hauteur verticale de la cuve de réacteur.

8. Bioréacteur à semi-conducteur à échelle de production selon la revendication 1, comprenant en outre un filtre d'entrée d'air communiquant, de manière opérationnelle, avec au moins l'une des ouvertures.

9. Bioréacteur à semi-conducteur à échelle de production selon la revendication 1, comprenant en outre un filtre de sortie d'air communiquant, de manière opérationnelle, avec au moins l'une des ouvertures.

10. Bioréacteur à semi-conducteur à échelle de production selon la revendication 1, comprenant en outre une étiquette d'identification lisible par machine.

11. Système de bioréacteur à semi-conducteur à échelle de production comprenant :
une pluralité de bioréacteurs à semi-conducteur à échelle de production selon la revandication 1 ;
une station de fermentation comprenant une pluralité de conduites d'alimentation en air et de conduite d'évacuation d'air, la station de fermentation et la pluralité de bioréacteurs étant mutuellement adaptées pour raccorder de manière opérationnelle et réversible chacun de la pluralité de bioréacteurs à au moins une conduite d'alimentation en air et au moins une conduite d'évacuation d'air.

12. Système de bioréacteur à semi-conducteur à échelle de production selon la revendication 11, dans lequel :
la station de fermentation comprend une pluralité de sous-stations, chaque sous-station étant dimensionnée et configurée pour recevoir l'une de la pluralité de bioréacteurs à semi-conducteur à échelle de production.

13. Système de bioréacteur à semi-conducteur à échelle de production selon la revendication 11, dans lequel au moins certaines des sous-stations sont agencées dans une configuration empilée.

14. Système de bioréacteur à semi-conducteur à échelle de production selon la revendication 11, dans lequel chacun des bioréacteurs comprend en outre un moyen d'agitation disposé dans le compartiment intérieur de la cuve de réacteur dans une position située au-dessus de l'élément de plaque perforée.
